# EUROPEAN PATENT APPLICATION

(11) **EP 1 336 412 A1**
(43) Date of publication of application: **20.08.2003**
(21) Application number: 02003737.0
(22) Date of filing: 19.02.2002
(51) Int. Cl.: A61K 45/06

(54) **Combinations of an antibiotic and a murein hydrolase inhibitor**

(71) Applicant: Max-Planck-Gesellschaft zur Förderung der Wissenschaften e.V., 80539 München (DE)
(72) Inventor: Heidrich, Christoph, 56581 Melsbach (DE); Höltje, Joachim-Volker, 72108 Rottenburg (DE)
(74) Representative: Prüfer, Lutz H., Dipl.-Phys.

(57) **Abstract**

The invention relates to the field of antibiotics and is particularly useful for medical applications. In particular, the present invention relates to the combination of antibiotics with a means which provides an adjuvant, improving and/or enhancing effect for the antibiotic. The combination is provided in a therapeutic product or system, or in a pharmaceutical composition.

## Description

The present invention relates to the field of antibiotics and is particularly useful for medical applications. In particular, the present invention relates to the combination of antibiotics with a means which provides an adjuvant, improving and/or enhancing effect for the antibiotic. The combination is provided in a therapeutic product or system.

A large variety of antibiotics are known and applied in human and animal medicine for the control of bacteria. Resistance to antibiotics raised increasing problems in recent years (see "Antibiotic Resistance-Recent Developments and Future Perspectives", special edition "Biospektrum" (1997), Spektrum Akademischer Verlag, ISSN 0947-0867). A further challenging situation is based on the knowledge that Gram-negative bacteria are normally resistant towards antibiotics having a molecular weight larger than about 700 dalton due to the presence of an outer membrane that is not found in Gram-positive bacteria (see R.E.W. Hancock, "The bacterial outer membrane as a drug barrier", Trends in Microbiology 5: 37-42 (1997)). Conventionally, additives to antibiotics, like EDTA or polymyxin compounds, have been used to permeabilize the bacterial outer membrane and therefore to facilitate the control Gram-negative bacteria by the application of antibiotics having higher molecular weight R.E.W. Hancock and P.G.W. Wong in Antimicrob. Agents Chemother. 26, 48-52 (1984). However, due to the toxicity of EDTA and polymyxine, the application of this therapeutic approach is limited to exceptional cases.

The object of the invention is to improve the therapeutic concept of antibiotics.

The object is solved by the therapeutic product according to claim 1 and the pharmaceutical composition according to claim 8. In a further aspect, the present invention provides a process for obtaining substances which affect the permeability of the bacterial outer membrane according to claim 10. Preferred embodiments are defined in respective sub-claims.

According to the present invention, it was surprisingly found that when an antibiotic is combined with a means which is capable of inhibiting, or causes the inhibition of the cleavage or splitting of the bacterial murein septum, the bacterial outer membrane is rendered permeable. As a particular advantage, it was found that inhibiting the cleavage or splitting of the bacterial murein septum, Gram-negative bacteria became permeable to bulky antibiotics, for example antibiotics having a molecular weight of at least 500 dalton and even at least 700 dalton. Therefore, with the new concept of the present invention, highly potent antibiotics, the use of which normally has been limited to control Gram-positive bacteria, can now also be applied to Gram-negative bacteria. At the same time, inhibiting the cleavage or splitting of the bacterial murein septum can lead to the formation of chains of bacteria cells, because the separation of the daughter cells during cell division was often severely impaired. This leads to a general weakening of the bacterial cells and thus enhances the effectiveness of the therapeutic concept of the invention. Thus, the present invention does not only facilitate the application of antibiotics having a relatively high molecular weight of e.g. at least 500 dalton and particularly at least 700 dalton, but also improves the effectiveness of antibiotic therapeutic agents in general, including chemotherapeutic drugs and cytostatics, based on the effect of permeabilizing the bacterial outer cell membrane.

The present invention and the preferred embodiments and advantages features will be described in the following in further detail while referring to accompanying drawings.
- Fig. 1: is a schematic illustration, showing an example of inhibiting the cleavage or splitting of the bacterial murein septum by means of controlling the enzyme activities of amidases, lytic transglycosylases and/or endopeptidases;
- Fig. 2: shows the result of inhibiting the cleavage or splitting of the bacterial murein septum as an electron microscopical view of a thin section of a plasmolysed E.coli cell;
- Fig. 3: schematically shows a possible structure of a bacterial cell with an adjacent, non-separated cell being connected by an uncleaved septum;
- Fig. 4: is a graph showing the effect of deoxycholate on the growth control of E.coli, which had been treated that the cleavage or the bacterial murein septum is inhibited;
- Fig. 5: is a graph showing the effect of vancomycin on the growth control of E.coli, which had been treated that the cleavage of the bacterial murein septum is inhibited; and
- Fig. 6: shows in an electron microscopical view the effect of lysozyme on E.coli, which had been treated that the cleavage of the bacterial murein septum is inhibited.

The cleavage or splitting of the bacterial murein septum can be affected and thereby inhibited by various means, which are described in the following.

A suitable approach of inhibiting the cleavage of the bacterial murein septum is based on genetic means, i.e. genetically modifying the bacteria to be controlled such that the murein septum cleavage is inhibited. Based on the construction of corresponding deletion mutants, it was found that murein hydrolases are essentially involved in cell separation following cell division. Therefore, murein hydrolases, notably amidases, lytic transglycosylases and endopeptidases, which are involved in the cleavage of the murein septum turned out to be the appropriate targets for the selected means. Accordingly, genetically modifying means which lead to an inactivation of at least one murein hydrolase which is involved in the cleavage of the murein septum is a suitable means to be combined with an appropriate antibiotic in accordance with the present invention.

Different murein hydrolases exist (G.D. Shockman and J.-V. Hoeltje (1994) Microbial peptidoglycan (murein) hydrolases, p. 131-166. in: Bacterial Cell Wall (J.-M. Ghuysen and R. Hakenbeck eds., Elsevier Science Publishing, Amsterdam, The Netherlands). Preferably, at least one enzyme of the group of enzymes consisting of amidases, lytic transglycosylases and endopeptidases are affected by the means of the present invention. The enzymes may be affected individually, but are affected preferably in combination by inhibiting or inactivating several species or sub-species of enzymes falling under the aforementioned group. As the preferred target of amidases, N-acetylmuramyl-L-alanine amidases are mentioned.

In a preferred embodiment of the present invention, the means which is capable of inhibiting, or causes the inhibition of a cleavage of the bacterial murein septum is an inhibitor of the at least one murein hydrolase which have been defined as suitable targets. Especially, an inhibitor of an enzyme of the group of enzymes consisting of amidases, lytic transglycosylases and endopeptidases. As in the case of genetic modification means, the inhibitor exhibits an inhibiting effect on at least one murein hydrolase, but preferably represents a general inhibitor of several murein hydrolase enzymes among the group of amidases, lytic transglycosylases and endopeptidases. Again, the preferred targets for the inhibitor against amidase enzymes are N-acetylmuramyl-L-alanine amidases. More than one inhibitor may be used in combination for affecting a variety of murein hydrolases.

Various compounds or classes of compounds mentioned in the following, which are known to have inhibitory effects on murein hydrolases like amidases, lytic transglycosylases and endopeptidases, may be used in combination with an antibiotic in accordance with the present invention:

### Amidase inhibitors:

lipoteichoic acids (LTAs) and derivatives thereof (see W. Fischer et al. in J. Bacteriol. 146 (2), 467-475 (1981); and J.V. Holtje and A. Tomasz in PROC. NATL. ACAD. SCI. USA 72 (5), 1690-1694 (1975);
free cholin and deoxycholate (E. Diaz et al. in J. Bacteriol. 174 (17), 5508-5515 (1992)
N-acetylmuramyl-L-alanyl-D-glutamyl-meso-diaminopimelic acid (H. Wolf, Watz and S. Normark in J. Bacteriol. 128 (2), 580-586 (1976)
phosphatidyl glycerol, optionally with further additives (E. Vanderwinkel et al. in Biochem. Biophys. Acta 913 (2), 238-244 (1987); and E. Vanderwinkel and M. De Vlieghere in Biochem. Biophys. Acta 838 (1), 54-59 (1985).

### Lytic transglycosylase inhibitors:

bulgecins (O-sulfonated glyco peptides) (A.M. Thunnisson et al. in Biochemistry 34 (39), 12729-12737 (1995))
hexasaccharides, especially hexa-N-acetylchitohexaose (A. K. Leung et al. in Biochemistry 40 (19), 5665-5673 (2001)
Compounds designed on the basis of the crystallographic
inhibitor-binding study as described by A.N. Thunnissen et al. in Nature 367 (6465), 750-753 (1994).

### Endopeptidase inhibitors:

p-hydroxymercuribenzoate (T. Burgogne et al. in Int. J. Biochem. 24 (3), 471-476 (1992)).

In a further embodiment of the invention, another means capable of effecting the inhibition of the cleavage of the bacterial murein septum are antibodies raised against at least one murein hydrolase enzyme referred to above in a manner that the corresponding enzyme is inactivated or inhibited. For example, an antibody raised against N-acetylmuramyl-L-alanine amidases (see e.g. M. Sugai et al. in J. Bacteriol. 179 (9), 2958-2962 (1997)) may be used.

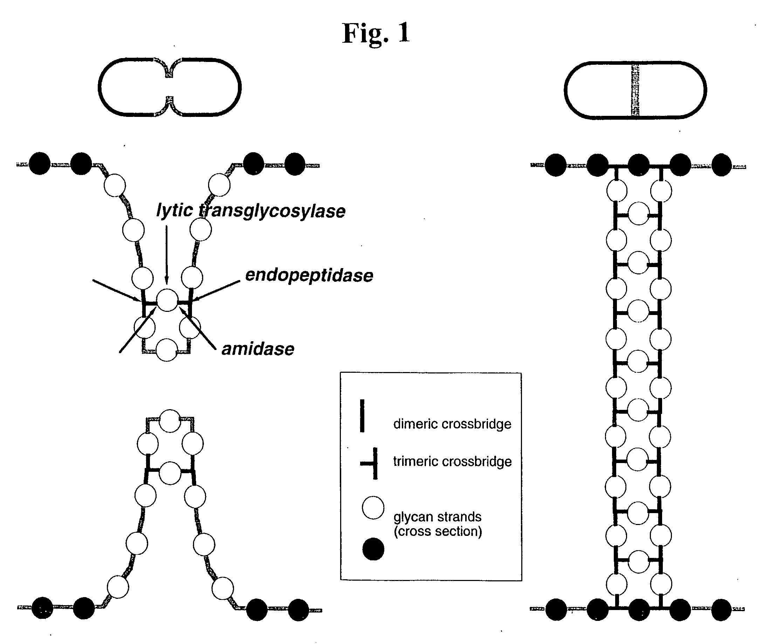

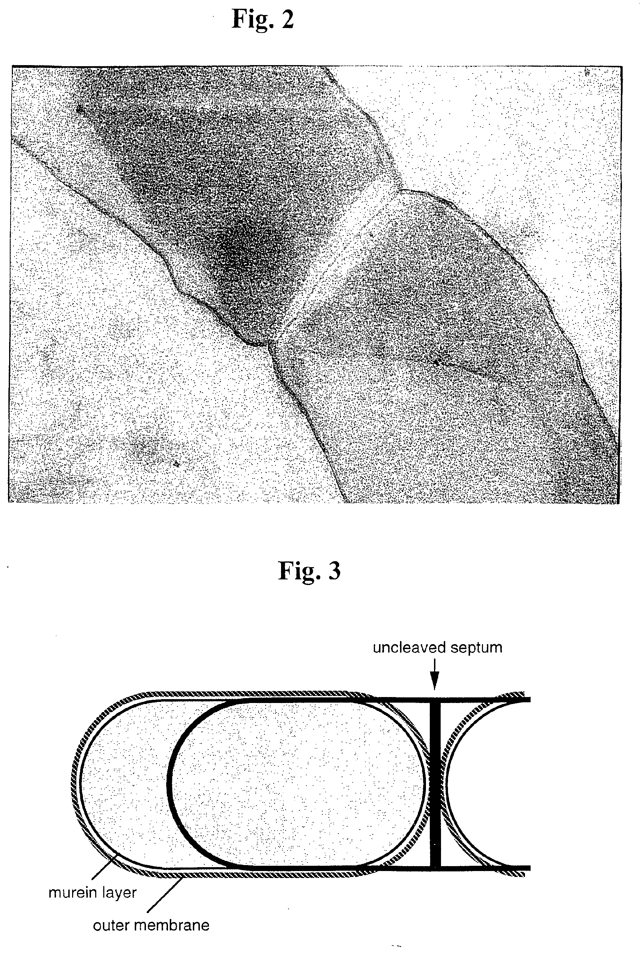

The mechanism which is involved in inhibiting the cleavage of the bacterial murein septum and thus in rendering the bacterial outer membrane permeable is described by referring to Figures 1 and 2. As schematically shown on the left side of Fig. 1, the normal situation is characterized by the action of murein hydrolases including amidase, lytic transglycosylase and endopeptidase on dimeric or trimeric crossbridges of glycan strains at the site of cell division (filled circles show glycan strains of the normal murein layer, whereas open circles show glycan strains of the septum). In order that separated daughter cells can be formed, the septum must be cleaved by the murein hydrolases. When the murein hydrolases involved in septum cleavage are inhibited or inactivated as described above, the septum is not cleaved, as shown on the right side of Fig. 1. Therefore, the cell division into daughter cells is deteriorated, and chains of bacteria cells connected by the septa of some length tend to be formed, for example having two or more or six or more and up to e.g. 100 cells per chain. Fig. 2 shows an electron microscopic view of a thin section of a plasmolized E.coli cell where the cleavage of the bacterial murein septum was inhibited by genetic means. The uncleaved septum is clearly visible. A schematic illustration of a possible structure of a Gram-negative bacterial cell having a murein layer and an outer membrane, which cell is connected to an adherent bacterial cell in a status having an uncleaved septum, is shown in Fig. 3.

With the permeabilisation of the outer membrane of the cell of Gram-negative bacteria, especially towards antibiotics having relatively high molecular weights, the therapeutic concept of the present invention differs from a direct lytic mechanism acting on the cell membrane as a response to cell wall synthesis inhibitors like penicillin. According to the invention, the effects of the antibiotic agent are enabled, enhanced and/or improved due to their ability to penetrate through the bacterial outer membrane as a consequence of the septum cleavage inhibition.

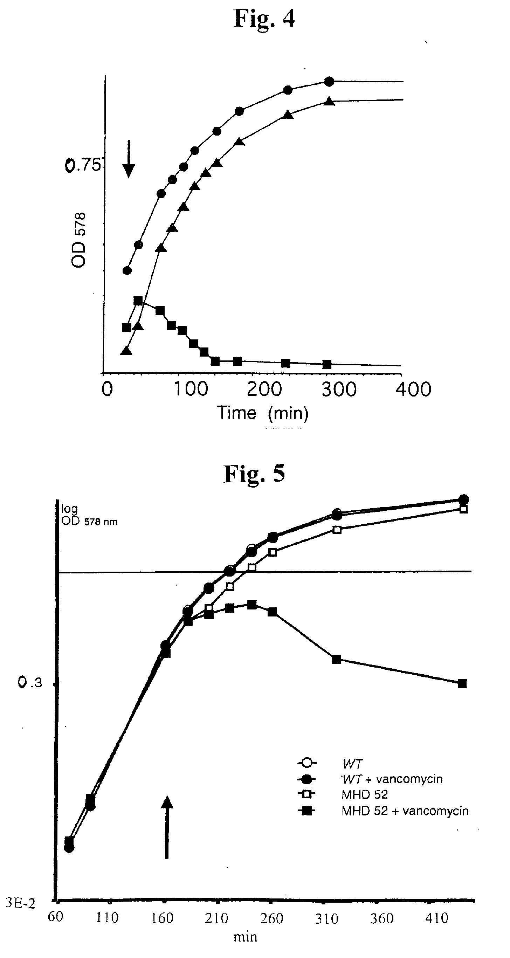

For example, Fig. 4 shows the growth inhibiting effect of deoxycholate (filled squares, the arrow indicates the time of adding deoxycholate). On the other hand, a treatment with deoxycholate in a system where the cleavage of the bacterial murein septum is not inhibited shows no growth inhibiting effect (filled circles). Furthermore, treatment by the mere toxic agent crystal violet had an insignificant effect (filled triangles).
Fig. 5 shows the growth controlling effect on E.coli of vancomycin in a system of inhibiting the cleavage of the bacterial murein septum (filled squares, the arrow indicates the time of adding vancomycin). Inhibiting the cleavage of the bacterial murein septum alone generated an almost normal growth (open squares). Treatment by vancomycin alone, i.e. without inhibiting the cleavage of the bacterial murein septum (see filled circles), generated normal growth (open circles). Following the inhibition of the cleavage of the bacterial murein septum, thereby rendering the outer cell membrane permeable, a significant increase in the sensitivity of bacterial cells was also demonstrated towards detergents such as Triton X100 and high molecular weight antibiotics other than vancomycin, such as mersacidin and even ramoplanin which has a molecular weight of 2.550. Even the sensitivity of the chain forming bacteria towards lysozyme was increased, as demonstrated by the finding that lysozyme induced lysis in the absence of EDTA, which normally is essential to allow the permeation of the enzyme across the outer membrane.

The increased permeability of the bacterial outer cell membrane was found to be directly correlated with the formation of uncleaved or unclipped septa. Permeation of the outer membrane by an inhibition of the septum cleaving enzymes was not observed when septum formation was inhibited in advance by the addition of an antibiotic (aztreonam) that specifically inhibits this process. Thus, inhibitors of the hydrolases by themselves do not result in a permeabilisation.

Based on the effect achieved by means of inhibiting the cleavage of the bacterial murein septum, any antibiotic can be generally used according to the present invention, including chemotherapeutic drugs and cytostatic agents. Suitable antibiotics are compounds affecting cell wall biosynthesis such as penicillins, cephalosporins, vancomycin, cycloserin etc., compounds affecting the cytoplasm membrane such as polyen-antibiotics and polypeptide-antibiotics, compounds affecting the transcription (rifampicin, actinomycins etc.), the translation (tetracylins, chloramphenicol, streptomycin, erythromycin, lincomycin etc.) and the replication (novobiocin, mitomycin etc.) of the cell, and compounds affecting the respiratory chain such as antimycin, valinomycin etc. Due to the effect of rendering the bacterial outer membrane permeable, antibiotic compounds having a molecular weight of at least 500, in particular at least 700 dalton can be advantageously used for the control of Gram-negative bacteria. Therefore, high molecular weight antibiotics which proofed to be highly potent against Gram-positive bacteria can now be also used for the control of Gram-negative bacteria. Examples of high molecular weight antibiotics of this type are vancomycin, bacitracin, erythromycin etc.. When combined with the inhibition of the cleavage of the bacterial murein septum, it was shown that high molecular weight antibiotics or chemotherapeutic compounds effectively control the growth of Gram-negative bacteria.

The present invention is not only highly valuable in reducing the bacterial resistance towards antibiotics and in extending the applicability of previously known antibiotics, such as the possibility of applying antiobiotics against Gram-positive bacteria also to Gram-negative bacteria. Moreover, since the therapeutic concept of the present invention leads to an increased permeability of the bacterial outer cell wall, the development of further, yet unknown antibiotics or chemotherapeutic drugs is facilitated. The present invention is therefore beneficially applicable to the field of medical treatment of humans and animals.

The therapeutic product according to the present invention may be formulated in a pharmaceutical composition with a pharmaceutically acceptable carrier. The active ingredients, most suitably embodied by the combination of the antibiotic and the at least one murein hydrolase inhibitor, are contained in the pharmaceutical composition in therapeutically effective amounts to produce an antibiotic effect. Any suitable pharmaceutically acceptable carrier can be used, for example those which have been conventionally used with antibiotic drugs. Furthermore, any suitable dosage form and mode of administration can be used.

The present invention further provides a process for obtaining substances which affect the permeability of the bacterial outer membrane, which process comprises the steps of:
- providing a substance to be tested,
- performing an enzymatic reaction of which involves at least one enzyme selected from the group consisting of murein hydrolases in the presence of the substance to be tested, and
- selecting that substance which exhibits an inhibitory action on said enzymatic reaction.

In order to provide further means and therapeutic agents to be combined with the antibiotic agent according to the present invention, the substance which has been selected as the result of the above process should have the property of inhibiting the cleavage of the bacterial murein septum. The enzymatic reaction is most suitably performed with an enzyme of the group of enzymes consisting of amidases, lytic transglycosylases and endopeptidases, which group of enzymes have been described above. Preferably, that substance is selected among the tested ones which exhibits a general inhibitory action on several types of enzymes among this group of amidase, lytic transglycosylase and endopeptidase enzymes. The substance to be tested may be derived from a chemical library, a natural substance library or from a system of combinatory chemistry. When the process is carried out, parallel negative (without a substance to be tested) and positive (with a known murein hydrolase inhibitor) control runs can be performed.

The substance which is obtained by this process is useful as a therapeutic agent in combination with an antibiotic.

The present invention is described in further detail by the following, non-limiting examples.

### Example 1:

Bacterial strains, bacteriophages and plasmids: Deletion mutants were constructed from Escherichia coli MC1061 (M.J. Casabadan and S.N. Cohen in J. Mol. Biol. 138: 179-207 (1980)) and E. coli CS203 (D.E. Nelson and K.D. Young in J. Bacteriol. 182, 1714-1721 (2000)), respectively. The general cloning vector was pBluescriptII SK+ (Stratagene, La Jolla, CA).
Either the Kohara miniset λ library (A. Noda et al. in Biotechniques 10, 476-477 (1991)) was used for transfer of the plasmid encoded gene deletions into the chromosome or vector pMS7, a sacB containing modification of pMAK700 (C.M. Hamilton et al, in J. Bacteriol. 171, 4617-4622 (1989), was employed for the generation of chromosomal gene knockouts (S. Kulakauskas in J. Bacteriol. 173, 2633-2638 (1991)). The kanamycin resistance determinant was taken from pUC4K (Pharmacia, Uppsala, Sweden), the chloramphenicol resistance marker from pBCSK+ (Stratagene, La Jolla, CA) and the tetracyclin resistence gene from pBR322 (New England Biolabs, Beverly, MA).

### Growth conditions:

Bacteria were cultivated aerobically at 37° C in LB medium (J.H. Miller, Experiments in Molecular Genetics, Cold Spring Harbor Laboratory, Cold Spring Harbor (1972)). Agar plates contained 1.5 % agar (Gibco BRL, Paisley, Scotland). Growth was monitored by optical density readings at 578 nm in an Eppendorf photometer (Eppendorf, Hamburg, Germany). Antibiotics were used at the following concentrations: 50 (µg/ml ampicillin (amp), 50 (µg/ml kanamycin (kan), 20 (µg/ml chloramphenicol (cm) and 12.5 µg/ml tetracyclin (tet).

### DNA manipulations and PCR:

Standard techniques were used to manipulate DNA, and E. coli was transformed using the modified calcium chloride procedure (J. Sambrook et al., Molecular cloning: a laboratory manual. Cold Spring Harbor, N.Y., Cold Spring Harbor Lanoratory Press (1989)). Restriction endonucleases were purchased from Roche Diagnostics (Mannheim, Germany), oligonucleotides were obtained from MWG-Biotech (Ebersberg, Germany).
PCR was performed on a MJ Research Inc. PTC-200 (Biozym, Oldendorf, Germany) using 0.5 U/25 µl Powerscript polymerase (PAN systems GmbH, Nürnberg, Germany) to create products with deleted ORFs or using 0.7 U/25 µl Taq polymerase (MBI Fermentas, Vilnius, Lithuania) to screen for successful chromosomal gene deletions.
Each primer was added to a final concentration of 0.5 µM. After initial denaturation for 3 min at 92° C, touchdown PCR (R.H. Don et al. in Nucl. Acid. Res. 19, 4008 (1991)) was performed at 72° C with 1 min of annealing, 1-3 min of extension, depending on the distance of the primer binding sites, and 0.5 min of denaturation at 92° C. The annealing temperature was initially 52° C, and then decreased in 12 cycles by 0.5° C each cycle. Finally the annealing temperature was 46° C for another 12 cycles.
The primers used to create plasmid encoded gene deletions are listed in Table 1. The primer sequences in the listed order correspond to SEQ ID Nos 1 to 25.

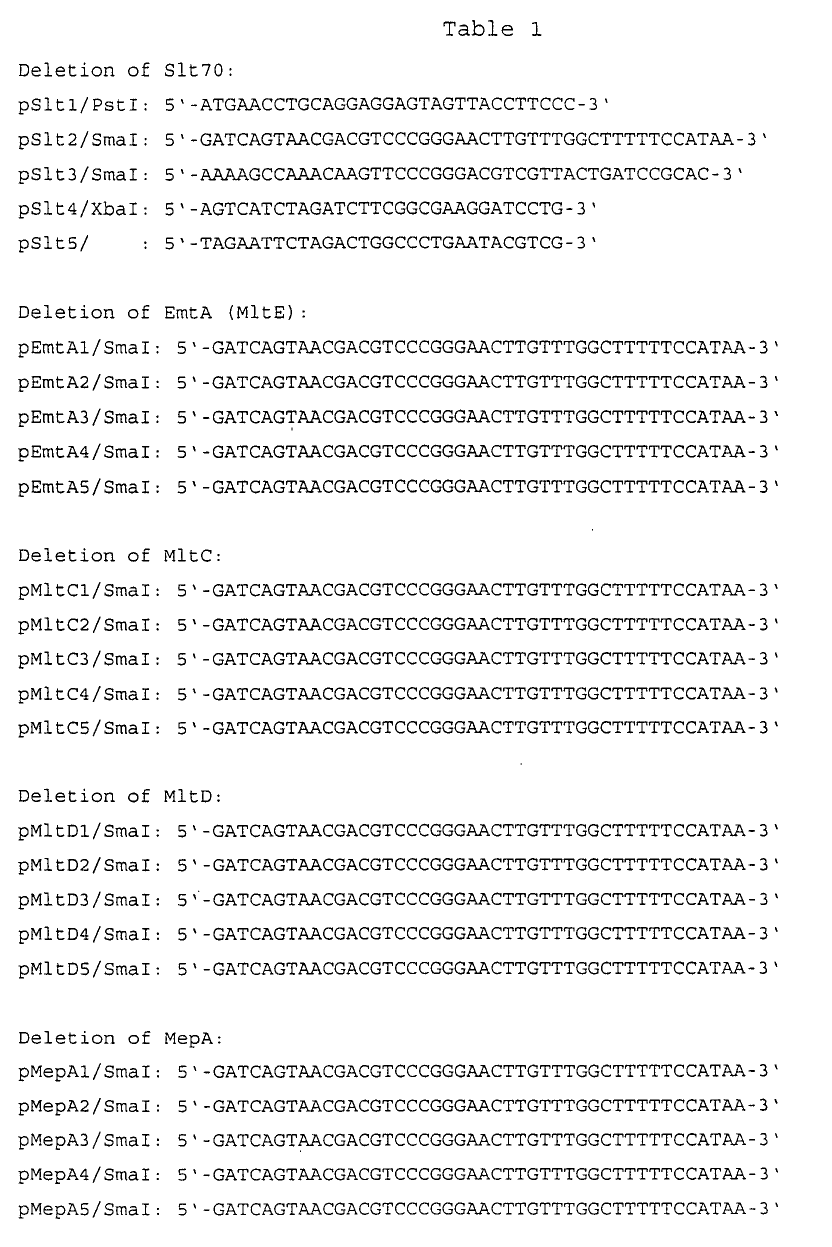

Each respective primer pair marked with numbers 1,2 and 3,4 resulted in PCR products upstream and downstream of the selected ORF containing overlapping sequence ends. The probes were mixed giving the final PCR product in a third PCR using primer 1 and 4. The DNA fragment was ligated with pBluescriptII SK+ and used to transform E. coli MC1061.

The deletion strategy of AmiA, B, C has been described by C. Heidrich et al. in Mol. Microbiol. 41, 167-178 (2001), the deletion of MltA, MltB has been reported by Lommatzsch et al. in J. Bacteriol. 179: 5465-5470 (1997)) and the deletion of PBP4 and 7 has been published by Nelson and Young (see above).

### Construction of deletion mutants:

A modification of the gene exchange method of Kulakauskas et al. (see above) was used to construct deletions of the coding regions of the murein hydrolase genes. First, the respective gene deletion carried by pBluescriptII SK+ was transferred to the modified vector pMS7. Optional a selection marker was introduced at the position of the missing gene employing SmaI restriction sites created by the primers 2 and 3, respectively. For cointegrate formation, growth in liquid culture at 28° C for at least 6 h was followed by plating on LB/amp plates and incubation at 42° C. Successful cointegrates were found by PCR with the primer combination T7 primer (NEB, Beverly, MA)/ pAmiX5 with X = A or B. Several of the cointegrates were incubated at 28° C in liquid culture for at least 6 h and finally plated on LB plates at 37 °C supplemented with 4 % sucrose, which is harmful for the pMS7 carrying strains because of the gene locus sacB and thus selects for replacement of the constructed gene deletion with the chromosomal wild type copy. Control for successful gene replacement was done by PCR with primers 1 and 5, respectively or pAmiCupup/pUC4Kupup in the case of amiC deletion. The transfer of an existing chromosomal deletion into different mutant strains was performed with bacteriophage P1 as described by Miller (see above).
The plasmid borne amiC deletion was transferred to the chromosome of E. coli MC1061 by transduction using Kohara phage 1233 as an intermediate employing the technique described in Kulakauskas et al. (see above).
Multiple deletions were achieved by different strategies: either by P1 transduction (see Miller above) of an existing chromosomal gene deletion to a dedicated host, which itself carried a gene deletion, if a resistence marker was introduced into the site of gene deletion, or by stepwise deletion of different genes using recombinant vector pMS7 repeatedly.
Murein analysis was done as described by Glauner et al. in J. Biol. Chem. 263, 10088-10095 (1988).

Multiple murein hydrolase deletion mutants were constructed to generate complete families of enzyme specificities, as summarised in Table 2, that is in lytic transglycosylases (MHD79), in amidases (MHD52) and endopeptidases (MHD31). Various deletions of different enzyme specificities were also combined. One mutant, MHD64, lacks 7 different murein hydrolases.

Cleavage of the murein septum, which is essential for separation of the daughter cells during cell division, was generally severely impaired. This gave rise to the formation of chains of cells sticking together by uncleaved murein septa (Fig. 2, Table 3). Mutant MHD75, deleted in mltC, mltD and mltE, was growing with 30% of the cells in short chains of 3-6 cells. Deletion of all known lytic transglycosylases in MHD79 resulted in growth of 80% of the cells in chains of 3-8 cells. Remarkably, the cells

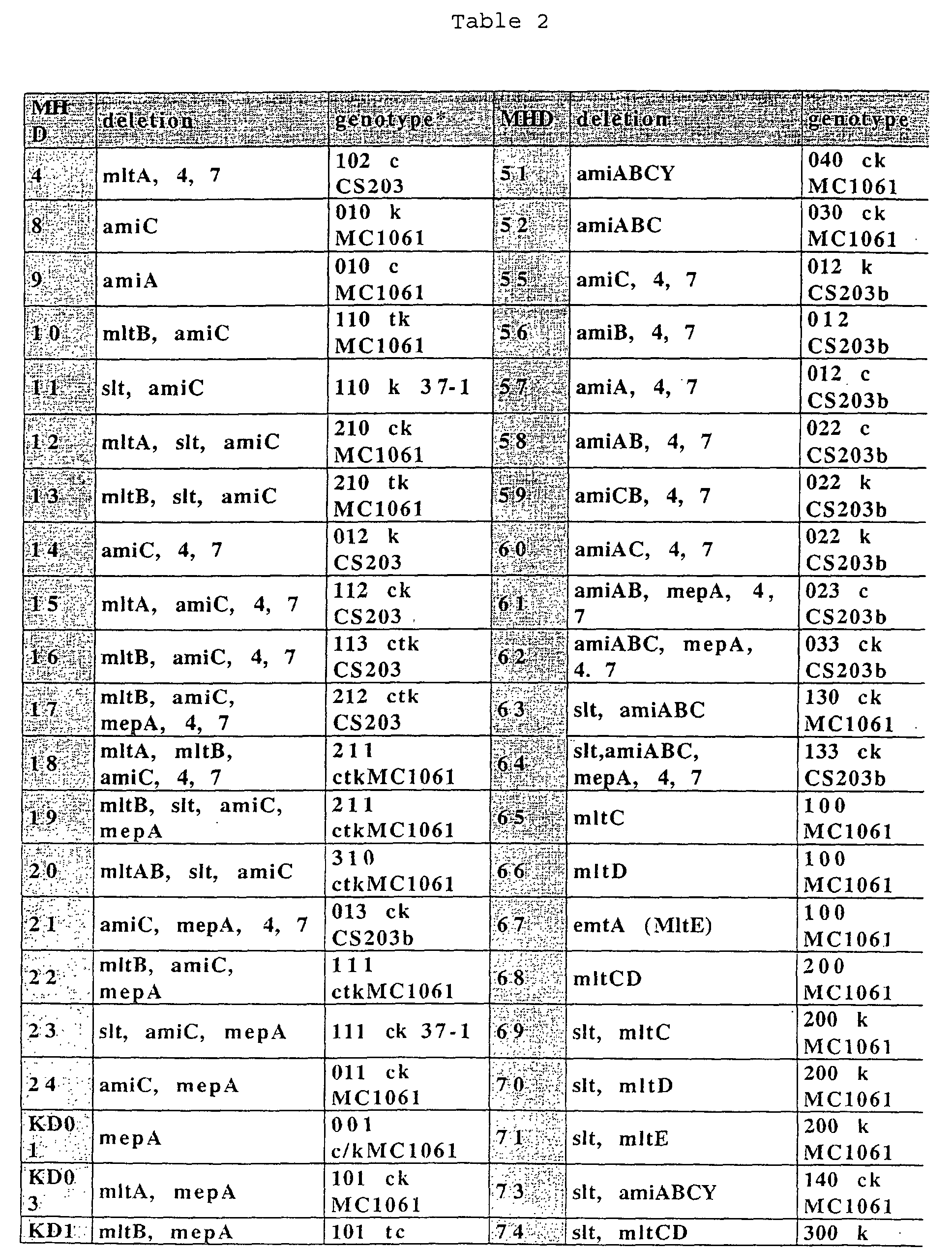

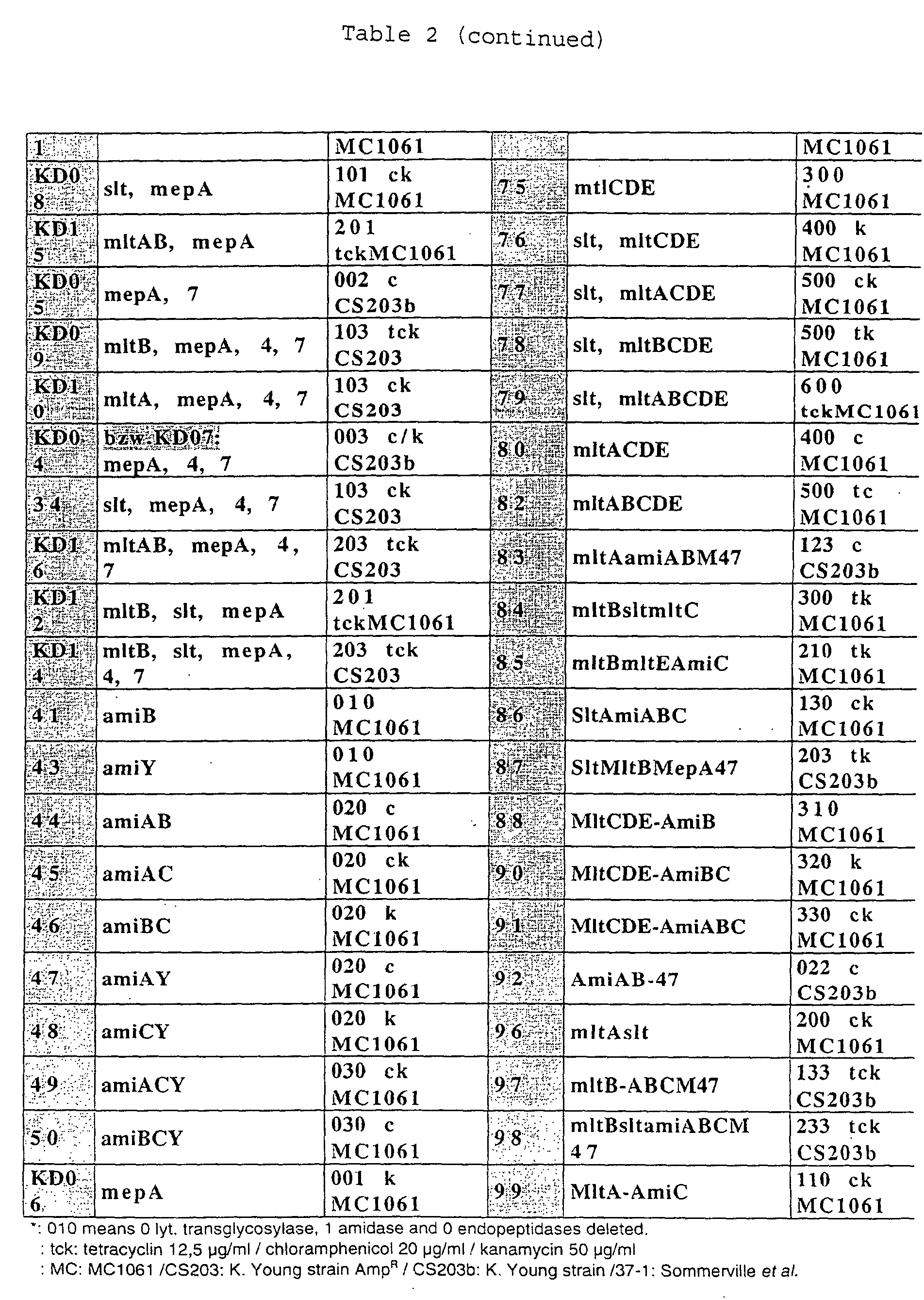

**Table 3**

| strain | deletion | resistance marker | % chains | cells/c hain |
|---|---|---|---|---|
| MHD 52 | AmiABC | cm, kann | 90 -100 % | 6 - 24 |
| MHD 33 | MepA,PBP4,7 | kann | - | - |
| MHD 79 | Slt, MltABCDE | cm, kann, tet | 30 - 50 % | 3 - 8 |
| MHD 6 2 | AmiABC, MepA,PBP4,7 | cm, kann | 100 % | 6 - 80 |
| MHD 6 4 | Slt,AmiABC, MepA,PBP4,7 | cm, kann | 100 % | 20 -100 |

had a slightly coccoidal shape indicating that cell elongation was impaired in this mutant. Predominantly N-acetylmuramyl-L-alanine amidases seem to be involved in splitting the septum. Deletion of one single amidase, AmiA, gives rise to the formation of short chains (3-4 cells/chain) and the length of the chains increases the more members of the amidase family are deleted. When the deletion of endopeptidases (MHD33) was combined with deletions in lytic transglycosylases and/or amidases the average number of cells in the chains was further increased. MHD62 which in addition to the amidases lacks the endopeptidases MepA, PBP4 and PBP7 forms chains of 6-80 cells. The mutant MHD64 that is deleted in all three amidases, all three endopeptidases and the major soluble lytic transglycosylase Slt70 formed the longest chains consisting of 20-100 cells.

Thus, it is demonstrated that inactivating the enzyme specificities of lytic transglycosylases, endopeptidases and amidases enables the inhibition of splitting the murein septum. Amidases, notably N-acetylmuramyl-L-alanine amidases, play a predominant role.

Increased permeability of the outer membrane was demonstrated by the following treatments:
E. coli MHD52 (deletion of AmiABC) and wild-type E. coli MC1061 were grown aerobically at 37°C in LB medium. At the indicated time point (arrow) deoxycholate (1 g/l) or crystal violet (1 mg/l), respectively, were added. See the results in Fig. 4 (filled circles: wild type E. coli plus deoxycholate; filled triangles: MHD52 plus crystal violet; filled squares: MHD52 plus deoxycholate).
Likewise, the cells were grown in the same manner, but at the indicated time point (arrow) vancomycin (20 µg/ml) was added. See the results in Fig. 5 (open circles: wild type E. coli; filled circles: wild type E. coli plus vancomycin; open squares: MHD52; filled squares: MHD52 plus vancomycin).

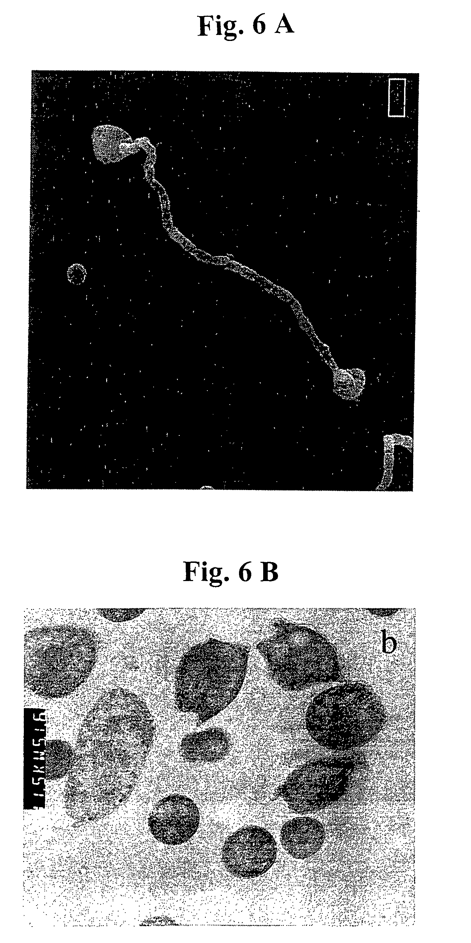

Furthermore, MHD52 mutant cells were incubated in LB at 37°C to OD578nm of 0.5-1.0 before addition of 10 µg/ml lysozyme. After 20 min, the cultures were prepared for electron microscopy. Fig. 6A shows, by scanning electron microscopy, the formation of barbell like ends of the chains treated with lysozyme. Ultrathin sections of these structures indicate that they are formed by fusion of several spheroplasts that are enclosed by a huge outer membrane envelope (Fig. 6B).

Thus, it was shown that the increase in permeability of the bacterial outer membrane is a result of the formation of unclipped murein septa. Inhibiting the cleavage of the bacterial murein septa affects the integrity of the outer membrane.

### Example 2:

Process for obtaining therapeutic substances involving an enzymatic test for N-acetylmuramyl-L-alanine amidase

A substance to be tested for its property of affecting the permeability of the bacterial outer membrane is added to an enzymatic reaction between N-acetylmuramyl-L-alanine amidase (Heidrich et al. in Mol. Microbiol. 41, 167-178 (2001)) and an isolated murein sacculus. 10 µl murein sacculi (0.5 mg murein/ml) is used as the enzyme substrate, the peptides of which were radioactively labeled by the incorporation of radioactive diaminopimelinic acid ([³H]-A₂pm) to a specific radioactivity of 1 x 10⁶ cpm/ml. Up to 20 µl of the amidase sample at a concentration of 0.5 mg/ml is added to the ([³H]-A₂pm) murein sacculi substrate. The reaction mixture is supplemented to 100 µl by 0.01 M Tris/HCl, 0.01 M MgCl₂, 0.1 mM DTE (pH 7.5). The enzymatic reaction is allowed to proceed for an incubation time of 30 minutes at 37°C. The enzymatic reaction is stopped by boiling the sample for 3 minutes. The sample is then cooled on ice. The portion of murein sacculi which has not been digested by the enzyme is then precipitated by adding 100 µl of 0.1 % cetyltrimethylammoniumbromid (CTAB), maintaining an incubation time of 20 minutes on ice. The sample is the centrifuged by an Eppendorf centrifuge, and the radioactivity in the supernatant (100 µl) is determined. The quantity of radioactivity measured in the supernatant is indicative for the amidase activity.

Enzymatic tests for other murein hydrolases are described by W. Kusser and U. Schwarz in Eur. J. Biochem. 103, 277-281 (1980) for lytic transglycosylases, and by T. Romeis and J.-V. Hoeltje in Eur. J. Biochem. 224, 597-604 (1994) for endopeptidases.

## Claims

1. A therapeutic product comprising an antibiotic and means which is capable of inhibiting the cleavage of the bacterial murein septum.

2. The product according to claim 1, **characterized in that** said means which causes the inhibition of the cleavage of the bacterial murein septum is an inhibitor of at least one murein hydrolase.

3. The product according to claim 2, **characterized in that** the inhibitor is at least one inhibitor of an enzyme selected from the group of enzymes consisting of amidases, lytic transglycosylases and endopeptidases.

4. The product according to claim 2 or 3, **characterized in that** the inhibitor is an inhibitor of N-acetylmuramyl-L-alanine amidase.

5. The product according to any one of the preceding claims, **characterized in that** said antibiotic has a molecular weight of at least 500 dalton.

6. The product according to any one of the preceding claims for use in a medical treatment.

7. The product according to claim 6 for use in a medical treatment for the control of Gram-negative bacteria.

8. A pharmaceutical composition comprising a therapeutic product according to any one of claims 1 to 7 and a pharmaceutically acceptable carrier.

9. The pharmaceutical composition according to claim 8 for use as an antibiotic.

10. A process for obtaining substances which affect the permeability of the bacterial outer membrane, which comprises the steps of:
• providing a substance to be tested,
• performing an enzymatic reaction which involves at least one enzyme selected from the group consisting of murein hydrolases in the presence of the substance to be tested, and
• selecting that substance which exhibits an inhibitory action on said enzymatic reaction.

11. The process according to claim 10, wherein the enzymatic reaction is performed with an enzyme which is selected from the group consisting of amidases, lytic transglycosylases and endopeptidases.

12. A use of a substance as obtained according to the process of claim 10 or claim 11 as a therapeutic agent in combination with an antibiotic.


SEQUENCE LISTING

<110> Max-Planck-Gesellschaft zur Förderung der Wissenschaften e.V.
<120> Therapeutic Composition Comprising an Antibiotic
<130> M1-16293.6
<160> 25
<170> PatentIn version 3.1

<210> 1
<211> 31
<212> DNA
<213> synthetic
<400> 1
atgaacctgc aggaggagta gttaccttcc c 31

<210> 2
<211> 44
<212> DNA
<213> synthetic
<400> 2
gatcagtaac gacgtcccgg gaacttgttt ggctttttcc ataa 44

<210> 3
<211> 42
<212> DNA
<213> synthetic
<400> 3
aaaagccaaa caagttcccg ggacgtcgtt actgatccgc ac 42

<210> 4
<211> 30
<212> DNA
<213> synthetic
<400> 4
agtcatctag atcttcggcg aaggatcctg 30

<210> 5
<211> 30
<212> DNA
<213> synthetic
<400> 5
tagaattcta gactggccct gaatacgtcg 30

<210> 6
<211> 44
<212> DNA
<213> synthetic
<400> 6
gatcagtaac gacgtcccgg gaacttgttt ggctttttcc ataa 44

<210> 7
<211> 44
<212> DNA
<213> synthetic
<400> 7
gatcagtaac gacgtcccgg gaacttgttt ggctttttcc ataa 44

<210> 8
<211> 44
<212> DNA
<213> synthetic
<400> 8
gatcagtaac gacgtcccgg gaacttgttt ggctttttcc ataa 44

<210> 9
<211> 44
<212> DNA
<213> synthetic
<400> 9
gatcagtaac gacgtcccgg gaacttgttt ggctttttcc ataa 44

<210> 10
<211> 44
<212> DNA
<213> synthetic
<400> 10
gatcagtaac gacgtcccgg gaacttgttt ggctttttcc ataa 44

<210> 11
<211> 44
<212> DNA
<213> synthetic
<400> 11
gatcagtaac gacgtcccgg gaacttgttt ggctttttcc ataa 44

<210> 12
<211> 44
<212> DNA
<213> synthetic
<400> 12
gatcagtaac gacgtcccgg gaacttgttt ggctttttcc ataa 44

<210> 13
<211> 44
<212> DNA
<213> synthetic
<400> 13
gatcagtaac gacgtcccgg gaacttgttt ggctttttcc ataa 44

<210> 14
<211> 44
<212> DNA
<213> synthetic
<400> 14
gatcagtaac gacgtcccgg gaacttgttt ggctttttcc ataa 44

<210> 15
<211> 44
<212> DNA
<213> synthetic
<400> 15
gatcagtaac gacgtcccgg gaacttgttt ggctttttcc ataa 44

<210> 16
<211> 44
<212> DNA
<213> synthetic
<400> 16
gatcagtaac gacgtcccgg gaacttgttt ggctttttcc ataa 44

<210> 17
<211> 44
<212> DNA
<213> synthetic
<400> 17
gatcagtaac gacgtcccgg gaacttgttt ggctttttcc ataa 44

<210> 18
<211> 44
<212> DNA
<213> synthetic
<400> 18
gatcagtaac gacgtcccgg gaacttgttt ggctttttcc ataa 44

<210> 19
<211> 44
<212> DNA
<213> synthetic
<400> 19
gatcagtaac gacgtcccgg gaacttgttt ggctttttcc ataa 44

<210> 20
<211> 44
<212> DNA
<213> synthetic
<400> 20
gatcagtaac gacgtcccgg gaacttgttt ggctttttcc ataa 44

<210> 21
<211> 44
<212> DNA
<213> synthetic
<400> 21
gatcagtaac gacgtcccgg gaacttgttt ggctttttcc ataa 44

<210> 22
<211> 44
<212> DNA
<213> synthetic
<400> 22
gatcagtaac gacgtcccgg gaacttgttt ggctttttcc ataa 44

<210> 23
<211> 44
<212> DNA
<213> synthetic
<400> 23
gatcagtaac gacgtcccgg gaacttgttt ggctttttcc ataa 44

<210> 24
<211> 44
<212> DNA
<213> synthetic
<400> 24
gatcagtaac gacgtcccgg gaacttgttt ggctttttcc ataa 44

<210> 25
<211> 44
<212> DNA
<213> synthetic
<400> 25
gatcagtaac gacgtcccgg gaacttgttt ggctttttcc ataa 44